# EUROPEAN PATENT SPECIFICATION

(11) **EP 0 570 244 B1**
(45) Date of publication and mention of the grant of the patent: **11.02.1998**
(21) Application number: 93303756.6
(22) Date of filing: 14.05.1993
(51) Int. Cl.: C12P 21/00, C12N 9/48

(54) **Process for preparing peptides having a C-terminal proline amide**
Verfahren zur Herstellung von Peptiden mit C-terminalem Prolin-Amid
Procédé de préparation de peptides ayant un résidu proline amidé en position C-terminale

(30) Priority: 14.05.1992 JP 120959/92
(43) Date of publication of application: 18.11.1993
(73) Proprietor: SANKYO COMPANY LIMITED, Chuo-ku, Tokyo 103 (JP)
(72) Inventor: Tamaoki, Hidetsune, c/o Sankyo Company Limited, Iwaki-shi, Fukushima-ken (JP); Yoshikawa, Hiroji, c/o Sankyo Company Limited, Iwaki-shi, Fukushima-ken (JP); Yao, Yoshio, c/o Sankyo Company Limited, Tokyo 140 (JP); Ishikawa, Hirokazu, c/o Sankyo Company Limited, Tokyo 140 (JP); Kawaguchi, Junko, c/o Sankyo Company Limited, Tokyo 140 (JP)
(74) Representative: Gibson, Christian John Robert

(56) References cited:
- EP-A- 0 197 794

## Description

The present invention relates to a process for preparing peptides having a C-terminal proline amide.

The α-carboxyl group of the C-terminal amino acid residue is amidated in various eukaryotic proteins, resulting in the formation of an acid amide (-CONH₂). Examples of such proteins include certain types of peptide hormone and various types of growth factor originating from eukaryotic cells (referred to hereinafter simply as "peptides"). Amidation of these peptides is known to be essential for biological activity [Experimentaria (1976), 32, 246].

While these peptides are useful as therapeutic drugs against various diseases, they are, inherently, only present in vanishingly small amounts in living tissue, so that it is extremely difficult to prepare significant amounts of these peptides directly from such tissue. Thus, preparation of these peptides, and of human types in particular, has proven impossible until recently. With the progress of genetic engineering in recent years, it has now become possible to produce large amounts of these peptides in the form of recombinants.

In order to obtain large amounts of recombinant peptide, prokaryotic organisms, such as E. coli, are used. However, the necessary amidation cannot take place, because prokaryotic cells lack the necessary mechanism. Thus, in order to obtain the desired biologically active peptide, an amidation reaction must be carried out at the C-terminal α-carboxyl group. One method for achieving this relies on an extra amino acid being attached to the α-carboxyl group in the precursor.

The use of yeast carboxypeptidase Y, obtainable from baker's yeast for example, to amidate C-terminal proline, has previously been disclosed in EP-A-197794 [and also Tamaoki, H. et al., Ann Rep. Sankyo Res. Lab. (1986), 38, 73]. This process provides a method for amidation catalyzed by carboxypeptidase Y, in which the penultimate amino acid residue at the C-terminus of the substrate is proline, and wherein the C-terminus leaving group is a specific hydrophobic amino acid, but not a hydrophilic amino acid (such as glycine). Specific substrates for this reaction include peptides having, at their C-terminus, prolyl-leucine, prolyl-isoleucine, prolyl-valine, prolyl-phenylalanine and prolyl-alanine. The reaction is performed by treating the peptide substrate with carboxypeptidase Y in the presence of an excess of ammonia, or an ammonia-releasing substance, as a nucleophile, to give a peptide product with a C-terminal proline amide.

Carboxypeptidase Y catalyses two kinds of enzyme reaction. These are:
(1) a peptidase activity that indiscriminately and sequentially liberates amino acid residues from the C-terminal end of the peptide chain, and
(2) a transpeptidase activity in which the amino acid residue at the C-terminal is replaced by another amino acid residue or peptide.

The optimum pH for the peptidase activity described in (1) above is in the neutral region. The specificity of this reaction is extremely low.

The optimum pH for the transpeptidase activity is in the alkaline region, and the optimum pH can be provided, for example, by a concentration of ammonia in the region of 1 M. The peptide precursor is frequently only sparingly soluble in water, so that it is necessary to use an aqueous solvent system to prepare a solution of the precursor. Accordingly, a final concentration of precursor of the order of about 1 mM is generally used, in the presence of a concentration of 1 M amino acid, peptide, or ammonia, or higher, as a nucleophile.

Thus, it will be understood that the previously disclosed C-terminal amidation method using carboxypeptidase Y is dependent on the transpeptidase activity described in (2) above.

Genetic engineering techniques used to produce low molecular weight peptides generally involve the use of fusion proteins, wherein the peptide is coupled with another peptide or protein to provide chemical stability. In such methods, cDNA encoding the desired peptide is incorporated into an expression vector immediately adjacent DNA encoding another peptide sequence. The additional peptide sequence may serve to stabilise the desired peptide in the host cell, especially where the fusion product is not secreted, and/or the additional sequence may, for example, be a leader sequence, enabling the fusion product to be secreted from the host cell. The fusion protein must be cleaved to yield the desired peptide.

The cleavage reaction is generally performed using selected proteases. These enzymes cleave peptides only at specific sequences within the peptide. Accordingly, the linker portion between the two fused peptide sequences is typically engineered such that an amino acid sequence is generated which can be recognised and selectively cleaved by a selected protease. Because the protease will generally cleave a peptide bond in the middle of the recognition sequence, the resulting desired peptide will often possess one or more C-terminal amino acid residues in excess of the natural sequence after cleavage.

Thus, using genetic engineering to obtain the precursor for C-terminal amidated peptides is problematical. Not only can prokaryotes not perform the necessary amidation, but the precursor peptides will also usually be obtained in a form in which one or a plurality of amino acid residues are bound to the C-terminal end of the precursor peptide, after the cleavage reaction.

The prior art provides no method for the treatment of such peptides to obtain the desired peptide, other than by the use of a specific exopeptidase to remove the excess amino acid residues.

Clostripain is an example of a protease which can be used to cleave a fusion protein. The cleavage product resulting from the use of clostripain has a C-terminal prolyl-leucyl-arginine sequence, so that, in order for carboxypeptidase Y to be able to act, the arginine residue must be removed to expose a C-terminal prolyl-leucine. In order to remove the arginine, carboxypeptidase B can be used. The amidation reaction on the prolyl-leucine C-terminal can then be catalysed by carboxypeptidase Y.

On the other hand, where Staphylococcus aureus V8 protease is selected as the protease for use in the cleavage reaction, the C-terminal amino acid sequence of the peptide obtained is prolyl-leucyl-glutamic acid. This protease, therefore, cannot be used where an amidated end-product is required, as there is no method available to selectively remove only the glutamic acid residue to obtain the precursor peptide for the amidation reaction.

Although carboxypeptidase Y is known to possess peptidase activity, such activity is extremely non-specific, even in the neutral pH range, and this is further compounded by low activity in the alkaline pH range optimal for the transpeptidase activity. Thus, even if carboxypeptidase Y could be used as the necessary exopeptidase at the necessary alkaline pH, there would be a risk that, not only would the unwanted amino acids be cleaved, but that the peptide bonds between proline and leucine, for example, might also be cleaved.

Thus, as described above, the main problem with known methods of production of C-terminal amidated peptides is that it has been necessary to involve a separate process step to remove spurious amino acid residues prior to performing the C-terminal amidation reaction.

Accordingly, it is an object of the present invention to provide a simple process for both cleaving excess amino acids from and for subsequently amidating a precursor for a C-terminal amidated peptide.

Surprisingly, the present inventors have discovered that, contrary to expectations:
(a) carboxypeptidase Y does, in fact, have considerable peptidase activity even in the optimum pH range for catalysing the C-terminal amidation reaction; and
(b) the peptidase activity results only in minimal cleaving of peptide bonds either at the C-terminal side or the N-terminal side of proline under these conditions.

Thus, in a first aspect, there is provided the use of carboxypeptidase Y in the preparation, from a precursor, of a peptide product having a C-terminal amidated proline, wherein the precursor has a further peptide sequence consisting of at least two amino acid residues, not including proline, attached to the C-terminal proline and wherein, the further peptide sequence comprises a hydropholic amino acid attached to the proline by a peptide link, said use being in the presence of ammonia or an ammonia-releasing agent.

Thus, in a further aspect, the present invention provides a process for the preparation of a peptide product amidated at a C-terminal proline thereof, comprising contacting a precursor therefor with carboxypeptidase Y in the presence of ammonia or an ammonia-releasing agent to digest the precursor, the precursor having a C-terminal sequence of at least three amino acids starting with the proline, the sequence having no other proline residues, and wherein the amino acid adjacent the proline is a hydrophobic amino acid and is so selected that the action of the carboxypeptidase Y thereon, after digestion of the remainder of the sequence, will yield the proline amide.

In an alternative aspect, the present invention provides a process for the preparation of a peptide product amidated at a C-terminal proline, comprising cleaving a genetically engineered fusion protein precursor therefor to yield a cleaved precursor for the C-terminal amidated peptide and then contacting the cleaved precursor with carboxypeptidase Y in the presence of ammonia or an ammonia-releasing agent to yield the C-terminal amidated peptide, wherein the precursor has a sequence of at least two amino acids, other than proline, attached to a C-terminal thereof, the sequence comprising a hydrophobic amino acid attached to the proline by a peptide link.

According to a yet further aspect, there is provided a process for the preparation of a peptide product amidated at a C-terminal proline thereof, from a precursor therefor, the precursor comprising a derivative of the peptide wherein the C-terminal proline has, in place of the amide, a sequence of two or more amino acids having the formula -Z-W, wherein W represents one or more amino acids, the amino acids being the same or different, but not including proline, the process comprising contacting the precursor with carboxypeptidase Y in the presence of ammonia or an ammonia-releasing agent, under conditions in which the carboxypeptidase Y exhibits transpeptidase activity, thereby to cleave W, and wherein Z represents a group selected such that the action of the carboxypeptidase thereon after cleavage of the group W will yield the C-terminal proline amide, said group comprising a hydrophobic amino acid peptide linked to a C-terminal proline.

Accordingly, it is now possible to provide a one-step process for the production of C-terminal proline amidated peptides after cleavage, even where the cleavage product comprises a plurality of amino acid residues attached to the α-carboxyl of the C-terminal proline residue, using carboxypeptidase Y both to cleave unwanted amino acids and to selectively amidate the proline.

The resulting process made available by the present invention thus greatly simplifies the production of C-terminal amidated peptides by genetic engineering, by eliminating the separate stage of removing spurious amino acid residues from the precursor peptide obtained following the cleavage reaction. A peptide having a C-terminal proline amide can now be easily produced using genetic engineering techniques.

It will be appreciated that the present invention provides a simple process for the preparation of a peptide having a C-terminal amidated proline by the use of carboxypeptidase Y, wherein only carboxypeptidase Y is used, even where a plurality of amino acids is attached to the proline of the precursor.

Thus, it will be appreciated that the precursor is acting as a substrate for the enzyme, with amino acids being digested away until proline becomes the penultimate amino acid. Action of the enzyme on the residue immediately adjacent the proline residue at the C-terminal side thereof is then in accordance with the known method, and the desired C-terminal amidated peptide is obtained.

The preferred amino acids for positioning adjacent the proline at the C-terminal side, and which correspond to the group Z above, are the hydrophobic amino acids, and are especially leucine, isoleucine, valine, phenylalanine and alanine.

The ammonia is preferably present in molar excess relative to the enzyme substrate, or precursor, or is provided in molar excess by the ammonia-releasing substance. Ammonia-releasing substances may be in the form of a compound or compounds which, in situ, will release ammonia. A preparation of such compounds might include the treatment of a solution of an ammonium salt, such as ammonium sulphate, or preferably ammonium chloride, with an alkali. Suitable alkalis include the chlorides of sodium, potassium and lithium. In any event, the final pH of the ammonia preparation should be 8.0 or above, and preferably at least 8.5, for the transpeptidase activity of carboxypeptidase Y.

The substances comprising a peptide having a C-terminal proline amide, and the C-terminal amidated peptide products described above, will generally be referred to herein as "peptide products".

The peptide products of the present invention will necessarily comprise a C-terminal amidated proline, and usefully have a structure identical with that of a human peptide, such as the peptide hormones and growth factors referred to hereinabove, or structures sufficiently similar such that the peptide product possesses the desired physiological activity.

A particularly preferred peptide product is human calcitonin, although any other naturally occurring peptide having a C-terminal proline amide may also be made by the process of the present invention.

It will be appreciated that, while simple amidated peptides corresponding to natural products are preferred, the amidated product of the present invention may form part of a larger molecule, for example. Such a larger molecule may merely be in the form of a precursor for future cleavage into the active product, the precursor being in a form which cannot be attacked in the gut, for example, in the case of oral administration. It may also be that the final product consists of the peptide product in association with a larger molecule, such as a carrier protein, for example albumin, which may be used to raise antibodies. In any event, the present invention is not limited only to simple, amidated peptides. The only requirement of the precursor is that carboxypeptidase Y be able to act on that part containing the target proline in order to obtain the proline amide.

As the process of the present invention is an enzymatic process, it will be appreciated that the final yield will not generally approach 100%. However, yields in the region of 40 - 50% are readily attainable.

Because two types of enzymic activity are being relied upon, it will be appreciated that the enzymic process may also lead to the formation of side-products. For example, the exopeptidase activity of carboxypeptidase Y may lead to low level cleavage of the proline residue. For this reason, the reaction conditions should preferably be selected such as to minimise this possibility.

Side-products may also result from insufficient cleavage of the excess terminal amino acids, or failure to amidate the proline residue.

In order to reduce the quantity of side-products resulting from a failure to cleave all of the excess amino acids, it is desirable to keep excess residues attached to the proline residue of the precursor to a minimum. This may be achieved by engineering the linking region of the fusion protein such that a chosen protease can act immediately adjacent the target proline. Such engineering will often leave two or three residues in addition to the proline. If possible, the number of residues should be kept to two.

It will also be appreciated that the peptide products need not absolutely comply with the native peptide, where such is being copied. For example, deletions, insertions, mutations and inversions to the amino acid sequence may be convenient or desirable, and are envisaged as a part of the present invention. Likewise, it may be convenient or desired to take advantage of the degeneracy of the genetic code to engineer a sequence to produce the desired peptide which does not exactly correlate with that occurring in nature. This may be convenient where engineering a vector using restriction enzymes.

The optimum conditions for the processes of the present invention are generally as described in the following description of preferred embodiments.

The reaction may be carried out by mixing previously prepared aqueous solutions of substrate, carboxypeptidase Y and ammonia.

The aqueous substrate solution will preferably be prepared first. If the substrate is not very soluble in water, it may alternatively be dissolved in an aqueous dioxane or dimethyl sulphoxide solution.

To prepare a solution of carboxypeptidase Y, the enzyme may be dissolved in either water or aqueous ammonia solution, as desired. Carboxypeptidase Y is preferably used at a final concentration of about 1 to 5 µM where the final concentration of the substrate is about 0.1 to 0.3 mM [molecular weight of carboxypeptidase Y = ≃61,000; Hayashi, R. et al., J. Biol. Chem. (1973), 248, 2296]. If more carboxypeptidase Y is used then, while more amidated product will usually result, there is a risk that undesirable side-reactions will also occur.

The optimum pH at which the amidation reaction is carried out can vary according to temperature. For example, at 45°C, the optimum pH is about 8.4 to 8.5. At 37°C, the optimum pH is about 8.9 to 9.2. At a pH below about 8.0, the peptidase activity of carboxypeptidase Y is greater than the amidation activity, so that only very small quantities of amidated product will be obtained.

Although the amidation reaction can be carried out at about 25 to about 55°C, reaction yields are generally best in a reaction temperature range of about 45 to about 50°C. At temperatures below 25°C or above 55°C, the reaction yield tends to be significantly reduced.

The optimum reaction time when the reaction is carried out under the above conditions is preferably from about 0.5 to about 1 hour at a temperature of 45 to 50°C. Reaction times greatly in excess of 1 hour tend to result in decomposition of the product, thereby reducing yields.

The ammonia used for the amidation reaction is preferably provided in the form of a highly concentrated ammonia solution in distilled water. This solution can be adjusted to the target pH during preparation. A preferred method for the preparation of an ammonia solution is disclosed in EP-A-197794. Other suitable methods are exemplified below:
(1) Concentrated hydrochloric acid is added in small quantities to a 10 M solution of ammonia in distilled water, obtained by the addition of concentrated aqueous ammonia (29% v/v) to the water, in order to adjust the pH to 8.9, the solution being maintained at a temperature of 25°C. When raised to a temperature of 45°C, the prepared solution has a pH of 8.5, and an ammonia concentration of about 6.5 M;
(2) 326 g of ammonium chloride is added with stirring to 635 ml of distilled water which has previously been heated to about 60°C. After the ammonium chloride has completely dissolved, the temperature is lowered to 45°C, and 130 ml of 29% v/v aqueous ammonia, in small quantities, is then added with stirring, while maintaining the temperature at 45°C. This solution has a pH of about 8.4 to 8.5 in this state, and the ammonia concentration is 8.3 M. The resulting solution must be stored at about 45°C, as ammonium chloride precipitates at lower temperatures;
(3) 40 ml of distilled water is added to 578 ml of 29% v/v aqueous ammonia. The resulting solution is heated to 45°C and 571 ml of concentrated (12 N) aqueous hydrochloric acid, in small quantities, is added with stirring, while maintaining the temperature at 45°C. The prepared solution has a pH of about 8.4 to 8.5 in this state, with an ammonia concentration of 8.2 M. The resulting solution must be stored at about 45°C, as ammonium chloride precipitates at lower temperatures.

The final concentration of ammonia in the reaction mixture will be diluted when the ammonia solution is mixed with the aqueous solutions of peptide substrate and carboxypeptidase Y. However, the substrate and the enzyme may be dissolved in ammonia solution so as to avoid excessive dilution of ammonia in the reaction mixture, thereby helping to maximise the yield of amidation product. Typically, the solutions of substrate, enzyme and ammonia may be mixed in a ratio of about 1 : 1 : 8 v/v.

If desired, the relative proportions by volume of the substrate and enzyme solutions can be reduced, while the volume of ammonia solution can be increased. However, the ratio of volumes of solutions should be kept such that the substrate does not precipitate.

Identification and purification of the product obtained in the amidation reaction can be performed using known purification methods, such as ion exchange column chromatography and high performance liquid chromatography.

The present invention is described below in more detail by the non-limiting accompanying Examples.

In the Examples, CBZ represents benzyloxycarbonyl (carbobenzoxy), Ala represents alanine, Pro represents proline, Leu represents leucine, Lys represents lysine and Arg represents arginine. Unless otherwise specified, the starting materials were synthesised in accordance with the method of DeTar et al. [Journal of the American Chemical Society (1966), 88:5, 1024-1030].

### EXAMPLE 1

A 1.6 mM solution of the synthetic peptide substrate CBZ-Ala-Pro-Leu-Lys-OH was made up in 40 % v/v aqueous dioxane. 40% v/v aqueous dioxane was used, as the peptide substrate was only sparingly soluble in water. However, the subsequent amidation reaction was not adversely affected by the use of this solvent system.

Small quantities of concentrated aqueous hydrochloric acid were added to 10 M aqueous ammonia in order to adjust the pH to 8.9, the solution being maintained at a temperature of 25°C by ice-cooling during addition of the hydrochloric acid. The concentration of ammonia in the resulting solution was 6.4 M. Carboxypeptidase Y (manufactured by Peptide Research Laboratories) was dissolved in a portion of the resulting ammonia solution to a concentration of 1.7 mg/ml (25 µM).

75 µl of the substrate solution, 75 µl of the carboxypeptidase Y solution and 600 µl of the ammonia solution were then mixed. The final concentration of peptide substrate in the reaction solution was 0.16 mM, that of carboxypeptidase Y was 2.5 µM, and that of ammonia was 5.12 M. The resulting mixture was allowed to react at 50°C for 30 minutes. The reaction was then stopped by adding 150 µl of concentrated aqueous formic acid (99% v/v) and 0.38 g of urea. The final volume after the reaction had been stopped was 1155 µl.

A mixture of 500 µl of the final solution and 33 µl of the substrate solution was subjected to high-performance liquid chromatography using a reverse phase column (TSK™ gel, ODS-120T, 7.8 mm x 30 cm). The column was eluted with a linear concentration gradient of acetonitrile in 10 mM aqueous ammonium bicarbonate increasing from 15% to 45% at a flow rate of 2.5 ml/min for 40 minutes. Monitoring ultraviolet absorption at 225 nm, substrate was detected at 23.2 minutes ("minutes", as used in the present context, indicate retention time of a given fraction on the column), while CBZ-Ala-Pro-Leu-OH was detected at 16.3 minutes, and CBZ-Ala-Pro-NH₂ was detected at 22.2 minutes. Each of the yields from the initial amount of substrate was calculated from the ratio of the respective area obtained versus a standard on the same chromatogram. The yield of the amidation product CBZ-Ala-Pro-NH₂ was 43.5%, while those of the by-products CBZ-Ala-Pro-OH and CBZ-Ala-Pro-Leu-OH were 28.0% and 17.6%, respectively.

### EXAMPLE 2

A 1.6 mM solution of the synthetic peptide substrate CBZ-Ala-Pro-Leu-Arg-OH was made up in 40% v/v aqueous dioxane. Small quantities of concentrated aqueous hydrochloric acid were added to 10 M aqueous ammonia in order to adjust the pH to 8.9, the solution being maintained at a temperature of 25°C by ice-cooling during addition of the hydrochloric acid. The concentration of ammonia in the resulting solution was 6.4 M. Carboxypeptidase Y (manufactured by Peptide Research Laboratories) was dissolved in a portion of the resulting ammonia solution to a concentration of 1.7 mg/ml (25 µM).

75 µl of the substrate solution, 75 µl of the carboxypeptidase Y solution and 600 µl of the ammonia solution were then mixed. The final concentration of peptide substrate in the reaction solution was 0.16 mM, that of carboxypeptidase Y was 2.5 µM, and that of ammonia was 5.12 M. The resulting mixture was allowed to react at 50°C for 30 minutes. The reaction was then stopped by adding 150 µl of concentrated aqueous formic acid (99% v/v) and 0.38 g of urea. The final volume after the reaction had been stopped was 1155 µl.

A mixture of 500 µl of the final solution and 33 µl of the substrate solution was subjected to high-performance liquid chromatography using a reverse phase column (TSK™ gel, ODS-120T, 7.8 mm x 30 cm). The column was eluted with a linear concentration gradient of acetonitrile in 10 mM aqueous ammonium bicarbonate increasing from 15% to 45% at a flow rate of 2.5 ml/min for 40 minutes. Monitoring ultraviolet absorption at 225 nm, substrate was detected at 24.3 minutes. The amidation product CBZ-Ala-Pro-NH₂ was obtained in a yield of 39.3% from the initial amount of substrate.

### EXAMPLE 3

A 1.1 mM solution of the synthetic peptide substrate CBZ-Ala-Pro-Leu-(Ala)₃ was made up in 40% v/v aqueous dioxane. Small quantities of concentrated aqueous hydrochloric acid were added to 10 M aqueous ammonia in order to adjust the pH to 8.9, the solution being maintained at a temperature of 25°C by ice-cooling during addition of the hydrochloric acid. The concentration of ammonia in the resulting solution was 6.4 M. Carboxypeptidase Y (manufactured by Peptide Research Laboratories) was dissolved in a portion of the resulting ammonia solution to a concentration of 1.7 mg/ml (25 µM).

75 µl of the substrate solution, 75 µl of the carboxypeptidase Y solution and 600 µl of the ammonia solution were then mixed. The final concentration of peptide substrate in the reaction solution was 0.11 mM, that of carboxypeptidase Y was 2.5 µM, and that of ammonia was 5.12 M. The resulting mixture was allowed to react at 50°C for 30 minutes. The reaction was then stopped by adding 150 µl of concentrated aqueous formic acid (99% v/v) and 0.38 g of urea. The final volume after the reaction had been stopped was 1155 µl.

A mixture of 500 µl of the final solution and 33 µl of the substrate solution was subjected to high-performance liquid chromatography using a reverse phase column (TSK™ gel, ODS-120T, 7.8 mm x 30 cm). The column was eluted with a linear concentration gradient of acetonitrile in 10 mM aqueous ammonium bicarbonate increasing from 15% to 45% at a flow rate of 2.5 ml/min for 40 minutes. Monitoring ultraviolet absorption at 225 nm, substrate was detected at 18.3 minutes. The amidation product, CBZ-Ala-Pro-NH₂, was obtained in a yield of 36.4% from the initial amount of substrate.

### EXAMPLE 4

A 1.0 mM solution of the synthetic peptide substrate CBZ-Ala-Pro-Leu-(Ala)₅-OH was made up in 40% v/v aqueous dioxane. Small quantities of concentrated aqueous hydrochloric acid were added to 10 M aqueous ammonia in order to adjust the pH to 8.9, the solution being maintained at a temperature of 25°C by ice-cooling during addition of the hydrochloric acid. The concentration of ammonia in the resulting solution was 6.4 M. Carboxypeptidase Y (manufactured by Peptide Research Laboratories) was dissolved in a portion of the resulting ammonia solution to a concentration of 1.7 mg/ml (25 µM).

75 µl of the substrate solution, 75 µl of the carboxypeptidase Y solution and 600 µl of the ammonia solution were then mixed. The final concentration of peptide substrate in the reaction solution was 0.10 mM, that of carboxypeptidase Y was 2.5 µM, and that of ammonia was 5.12 M. The resulting mixture was allowed to react at 50°C for 30 minutes. The reaction was then stopped by adding 150 µl of concentrated aqueous formic acid (99% v/v) and 0.38 g of urea. The final volume after the reaction had been stopped was 1155 µl.

A mixture of 500 µl of the final solution and 33 µl of the substrate solution was subjected to high-performance liquid chromatography using a reverse phase column (TSK™ gel, ODS-120T, 7.8 mm x 30 cm). The column was eluted with a linear concentration gradient of acetonitrile in 10 mM aqueous ammonium bicarbonate increasing from 15% to 45% at a flow rate of 2.5 ml/min for 40 minutes. Monitoring ultraviolet absorption at 225 nm, substrate was detected at 20.1 minutes. The amidation product, CBZ-Ala-Pro-NH₂, was obtained in a yield of 30.3% from the initial amount of substrate.

### EXAMPLE 5

Human calcitonin is a peptide hormone consisting of 32 amino acids and having a C-terminal proline amide. The unamidated peptide can be prepared synthetically or by genetic engineering, and the present Example illustrates amidation of the C-terminal proline by the process of the present invention.

The precursor for carboxypeptidase Y, calcitonin-Leu-Arg, was prepared by each of two standard techniques (genetic engineering and chemical synthesis). More specifically, the methods of preparation of calcitonin-Leu-Arg were as follows.

### i) Genetic Engineering

An expression vector based on pUC18 was constructed, encoding a fusion protein comprising β-galactosidase and calcitonin. pUC18 [Norrander, J., et al., Gene (1986), 26, 101-106], which incorporates the β-galactosidase gene, was digested with Bbe I (Takara Co., Ltd.).

DNA encoding human calcitonin was then synthesised using a DNA synthesiser (Applied Biosystems, model 380B), with modifications to the sequence made according to codon usage in E. coli. The genetic sequence for human calcitonin, obtained by reverse transcription from mRNA, is disclosed by Lasmoles, F., et al. in EMBO (1985), 4, no. 10, 2603-2607. In addition to the sequence encoding calcitonin, suitable bases were added to each end for the purposes of ligation. At the 5' end, the ligation sequence encoded an additional arginine (CGCG on the non-coding strand, no extra bases on the coding strand). At the 3' end, the coding strand additionally encoded leucine and arginine, followed by a stop codon and a GCGC ligation sequence.

The synthesised DNA sequence was then ligated into the digested pUC18 vector, using T4 ligase (Takara Co., Ltd.). The resulting vector encoded a fusion protein coded for by: 230 base pairs of the β-galactosidase gene; the DNA sequence encoding human calcitonin; and a linker sequence coding for arginine, which provided a cleavage site for clostripain.

The expression vector thus constructed was then used to transform E. coli YA21 by the calcium chloride method (c.f. Molecular Cloning, A Laboratory Manual, supra).

The transformed E. coli was then cultured overnight at 37°C in 2 x YT medium [YT medium - 16 g bactotryptone (Difco), 10 g yeast extract (Difco) and 5 g NaCl in 1 l distilled water]. The transformant was then harvested by centrifugation and lysed by sonication, and the inclusion bodies were collected by centrifugation.

The inclusion bodies were subjected to sodium dodecyl sulphate polyacrylamide gel electrophoresis under reducing conditions, and were found to consist almost entirely of the desired fusion protein. Accordingly, the inclusion bodies were used, without further purification, for the next stage.

Solutions of each of clostripain (Sigma - C 7403) and the inclusion bodies were prepared in 4 M aqueous urea (pH 8.0). The solutions were then mixed to give an activity of 0.2 International Units per 1 mg fusion protein, and the digestion reaction was performed at 37°C for 3 hours to cleave the fusion protein at the carboxy side of each of the arginine residues, thereby leaving calcitonin-Leu-Arg.

The precursor, calcitonin-Leu-Arg, was isolated using a reverse phase HPLC column (YMC gel ODS-230-20 SM, I.D. 20mm x 300mm, column made by YMC Company in Japan). The HPLC was performed using eluant A (an aqueous solution of 0.1% trifluoroacetic acid) and eluant B (an aqueous solution of 70% acetonitrile and 0.1% trifluoroacetic acid), in a linear gradient (a 64 : 36 ratio of A : B for the first 10 minutes, then followed by a linear gradient, finishing at a ratio of 36 : 64 at 25 minutes).

### ii) Chemical Synthesis

Calcitonin-Leu-Arg was prepared using a chemical synthesiser (Applied Biosystems Japan, model 430A).

The support body used for the synthetic procedure was HMP (4-hydroxymethylphenoxymethyl) resin (Applied Biosystems Japan). The method used for the synthesis was in accordance with the synthesiser manufacturer's instructions, using Fmoc (9-fluorenylmethoxycarbonyl), HOBT (1-hydroxybenzotriazole) and NMP (N-methyl-pyrrolidone). The mean yield for each step was 98.5%. The Fmoc protecting group was removed using piperidine with the same equipment, also in accordance with the manufacturer's instructions.

The resulting peptide was then treated with a mixture of trifluoroacetic acid : ethanedithiol : anisole : ethylmethylsulphide (93 : 1 : 3 : 3) at room temperature for 3 hours, in order to eliminate any protecting groups and to cleave the peptide from the support resin.

The resulting peptide was precipitated by treatment with 7 volumes of ice-cooled diethyl ether, and the precipitate was collected using a 6 µm membrane filter (Toyo Roshi Kaisha, Ltd., part no. PF 060).

The suspended precipitate was dissolved in 2 ml trifluoroacetic acid, and 100 volumes of distilled water were then added to the solution obtained. The solution was lyophilised, and the resulting powder was dissolved in a 0.1% aqueous solution of trifluoroacetic acid. 1/200 volume of the resulting solution was then subjected to HPLC (YMC, AM-301, 4.6 mm x 100 mm). The solvent used was a 0.1% aqueous solution of trifluoroacetic acid in distilled water, and a linear gradient of aqueous acetonitrile, starting at 20% and finishing with 50%, was employed. The elution rate was 2 ml/min. Calcitonin-Leu-Arg was eluted at 6.4 minutes, giving a yield of 1 mg total protein.

Calcitonin-Leu-Arg, obtained as described above, was then reversibly S-sulphonated, in accordance with the method of R. David Cole [Methods in Enzymology, Vol. II, Ed. by Hirs, C.H.W., (1967), pp. 206 Academic Press, New York and London]. This procedure sulphonates disulphide bonds and prevents insolubilisation of the sample in the reaction solution.

326 g of ammonium chloride was dissolved in 635 ml of distilled water, with heating. 130 ml of 29% v/v aqueous ammonia was added in small qantities, while maintaining the temperature at 45°C, to prepare an ammonia solution. The resulting solution has a pH of 8.4 to 8.5, in this state, and an ammonia concentration of 8.3 M. The solution was kept at 45°C, as ammonium chloride precipitates at lower temperatures. A 3 mM solution of S-sulphonated human calcitonin-Leu-Arg was made up in 50% v/v aqueous dimethyl sulphoxide. Carboxypeptidase Y (manufactured by Peptide Research Laboratories) was dissolved in distilled water to a concentration of 12.2 mg/ml (0.2 mM).

20 ml of substrate solution and 200 ml of the prepared ammonia solution were mixed, after which 0.6 ml of the carboxypeptidase Y solution was immediately added. The final concentration of substrate in the reaction solution was 0.27 mM, that of carboxypeptidase Y was 5 µM and that of ammonia was 7.5 M. The resulting mixture was allowed to react at 45°C for 30 minutes. The reaction was then stopped by adding 44 ml of concentrated aqueous formic acid (99% v/v) and 110 g of urea. The final volume after stopping the reaction was 330 ml.

In order to confirm that the required reaction had taken place, 20 µl of the final reaction solution was subjected to analytical high-performance liquid chromatography using a reverse phase column (YMC, AM-312, 6 mm x 15 cm). The column was eluted using a linear concentration gradient of acetonitrile in 10 mM aqueous ammonium bicarbonate increasing from 24% to 29% for 16 minutes at a flow rate of 1 ml/min. By monitoring ultraviolet absorption at 225 nm, the amidation product, S-sulphonated human calcitonin, was detected at 14.3 minutes, while unchanged substrate was detected at 11.6 minutes. The presence of a small amount of by-products was also observed. The amount of each of the products was calculated from the ratio of the respective area found versus that of the standard. The yield of the amidation product was 45.5% of the initial amount of substrate.

In order to collect the reaction product, 250 ml of the solution obtained after the carboxypeptidase Y reaction was subjected to high-performance liquid chromatography using a reverse phase column (YMC gel, ODS-230-20-SM, 20 mm x 30 cm). This was eluted with a linear concentration gradient of acetonitrile in 10 mM ammonium bicarbonate increasing from 23% to 28% for 30 minutes at a flow rate of 16 ml/min. By monitoring ultraviolet absorption at 225 nm, the amidation product, S-sulphonated human calcitonin, was detected in a fraction at 22.5 minutes, and collected.

Reduced glutathione and ethylenediamine tetraacetic acid were added to the thus obtained fraction to final concentrations of 2 mM and 1 mM, respectively. After adjusting the pH to 8.5 with 29% v/v aqueous ammonia, the de-S-sulphonation reaction was allowed to proceed at room temperature for 2 hours, and 55 mg of human calcitonin was obtained.

## Claims

1. Use of carboxypeptidase Y in the preparation, from a precursor, of a peptide product having a C-terminal amidated proline, wherein the precursor has a further peptide sequence consisting of at least two amino acid residues, not including proline, attached to the C-terminal proline and wherein, the further peptide sequence comprises a hydrophobic amino acid attached to the proline by a peptide link, said use being in the presence of ammonia or an ammonia-releasing agent.

2. A process for the preparation of a peptide product amidated at a C-terminal proline thereof, comprising contacting a precursor therefor with carboxypeptidase Y in the presence of ammonia or an ammonia-releasing agent to digest the precursor, the precursor having a C-terminal sequence of at least three amino acids starting with the proline, the sequence having no other proline residues, and wherein the amino acid adjacent the proline is a hydrophobic amino acid and is so selected that the action of the carboxypeptidase Y thereon, after digestion of the remainder of the sequence, will yield the proline amide.

3. A process for the preparation of a peptide product amidated at a C-terminal proline, comprising cleaving a genetically engineered fusion protein precursor therefor to yield a cleaved precursor for the C-terminal amidated peptide and then contacting the cleaved precursor with carboxypeptidase Y in the presence of ammonia or an ammonia-releasing agent to yield the C-terminal amidated peptide, wherein the precursor has a sequence of at least two amino acids, other than proline, attached to the C-terminal thereof, the sequence comprising a hydrophobic amino acid attached to the proline by a peptide link.

4. A process for the preparation of a peptide product amidated at a C-terminal proline thereof, from a precursor therefor, the precursor comprising a derivative of the peptide wherein the C-terminal proline has, in place of the amide, a sequence of two or more amino acids having the formula -Z-W, wherein W represents one or more amino acids, the amino acids being the same or different, but not including proline, the process comprising contacting the precursor with carboxypeptidase Y in the presence of ammonia or an ammonia-releasing agent, under conditions in which the carboxypeptidase Y exhibits transpeptidase activity, thereby to cleave W, and wherein Z represents a group selected such that the action of the carboxypeptidase thereon after cleavage of the group W will yield the C-terminal proline amide, said group comprising a hydrophobic amino acid peptide linked to a C-terminal proline.

5. A process according to any preceding claim, wherein, in the precursor, an amino acid selected from leucine, isoleucine, valine, phenylalanine and alanine, is attached to the proline by a peptide link.

6. A process according to any preceding claim, wherein the ammonia is present in molar excess.

7. A process according to any preceding claim, wherein the carboxypeptidase Y is used at a final concentration of about 1 to about 5 µM and wherein the precursor is present at a final concentration of about 0.1 to about 0.3 mM.

8. A process according to any preceding claim, wherein the process is performed at a pH of 8.0 or above.

9. A process according to any preceding claim, wherein the process is performed at a temperature of between about 25 to about 55°C.

10. A process according to claim 9, wherein the process is performed at a temperature of between about 45 to about 50°C.

11. A process according to any preceding claim, wherein the process is performed for a period of from about 0.5 to about 1 hour.

12. A process according to any preceding claim, wherein the ammonia is provided in the form of a highly concentrated aqueous ammonia solution.

13. A process according to any preceding claim, wherein each of the precursor and the carboxypeptidase Y are provided in the form of an aqueous ammoniacal solution.

14. A process according to any preceding claim, wherein each of the precursor, the carboxypeptidase Y and the ammonia are provided in the form of an aqueous solution, and the solutions are mixed in a ratio of about 1 : 1 : 8.

15. A process according to any preceding claim, wherein the C-terminal amidated peptide product has a structure identical with that of human calcitonin, or has a structure sufficiently similar to that of human calcitonin as to possess human calcitonin physiological activity.

## Patentansprüche

1. Verwendung von Carboxypeptidase Y bei der Herstellung eines Peptidprodukts mit einem C-endständigen amidierten Prolin aus einer Vorstufe, wobei die Vorstufe eine zusätzliche Peptidsequenz aufweist, die aus mindestens zwei Aminosäureresten, die kein Prolin umfassen, besteht und an das C-terminale Prolin gebunden ist, und wobei die zusätzliche Peptidsequenz eine hydrophobe Aminosäure aufweist, die an das Prolin über eine Peptidbindung gebunden ist, wobei die Verwendung in Gegenwart von Ammoniak oder eines Ammoniak freisetzenden Mittels erfolgt.

2. Verfahren zur Herstellung eines Peptidprodukts, das an seinem C-terminalen Prolin amidiert ist, umfassend das Kontaktieren einer Vorstufe davon mit Carboxypeptidase Y in Gegenwart von Ammoniak oder eines Ammoniak freisetzenden Mittels zur Verdauung der Vorstufe, wobei die Vorstufe eine C-terminale Sequenz aus mindestens drei Aminosäuren, die mit Prolin beginnen, aufweist, wobei diese Sequenz keine weiteren Prolinreste enthält und wobei es sich bei der dem Prolin benachbarten Aminosäure um eine hydrophobe Aminosäure handelt, die so ausgewählt ist, daß die Einwirkung von Carboxypeptidase Y darauf nach Verdauung der restlichen Sequenz das Prolinamid ergibt.

3. Verfahren zur Herstellung eines Peptidprodukts, das am C-terminalen Prolin amidiert ist, umfassend das Spalten einer gentechnisch hergestellten Fusionsprotein-Vorstufe hierfür unter Bildung einer gespaltenen Vorstufe für das C-terminale amidierte Peptid und das anschließende Kontaktieren der gespaltenen Vorstufe mit Carboxypeptidase Y in Gegenwart von Ammoniak oder eines Ammoniak freisetzenden Mittels unter Bildung des C-terminalen amidierten Peptids, wobei die Vorstufe eine Sequenz aus mindestens zwei von Prolin abweichenden Aminosäuren, die an das C-terminale Ende davon gebunden sind, aufweist, wobei die Sequenz eine an das Prolin über eine Peptidbindung gebundene hydrophobe Aminosäure umfaßt.

4. Verfahren zur Herstellung eines Peptidprodukts, das an seinem C-terminalen Prolin amidiert ist, aus einer Vorstufe hierfür, wobei die Vorstufe ein Derivat des Peptids umfaßt, wobei das C-terminale Prolin anstelle des Amids eine Sequenz aus zwei oder mehr Aminosäuren der Formel - Z-W aufweist, worin W eine oder mehrere Aminosäuren bedeutet, die gleich oder verschieden sind, aber nicht Prolin umfassen, wobei das Verfahren folgendes umfaßt: das Kontaktieren der Vorstufe mit Carboxypeptidase Y in Gegenwart von Ammoniak oder eines Ammoniak freisetzenden Mittels unter Bedingungen, bei denen die Carboxypeptidase Y eine Transpeptidase-Aktivität aufweist, wodurch W gespalten wird, und Z eine Gruppe bedeutet, die so gewählt ist, daß die Einwirkung der Carboxypeptidase darauf nach Spaltung der Gruppe W ein C-terminales Prolinamid ergibt, wobei diese Gruppe eine an ein C-terminales Prolin über eine Peptidbindung gebundene hydrophobe Aminosäure umfaßt.

5. Verfahren nach einem der vorstehenden Ansprüche, wobei in der Vorstufe eine Aminosäure, die unter Leucin, Isoleucin, Valin, Phenylalanin und Alanin ausgewählt ist, über eine Peptidbindung an das Prolin gebunden ist.

6. Verfahren nach einem der vorstehenden Ansprüche, wobei das Ammoniak in molarem Überschuß vorhanden ist.

7. Verfahren nach einem der vorstehenden Ansprüche, wobei die Carboxypeptidase Y in einer Endkonzentration von etwa 1 bis etwa 5 µM verwendet wird und wobei die Vorstufe in einer Endkonzentration von etwa 0,1 bis etwa 0,3 mM vorhanden ist.

8. Verfahren nach einem der vorstehenden Ansprüche, wobei das Verfahren bei einem pH-Wert von 8,0 oder mehr durchgeführt wird.

9. Verfahren nach einem der vorstehenden Ansprüche, wobei das Verfahren bei einer Temperatur von etwa 25 bis etwa 55°C durchgeführt wird.

10. Verfahren nach Anspruch 9, wobei das Verfahren bei einer Temperatur von etwa 45 bis etwa 50°C durchgeführt wird.

11. Verfahren nach einem der vorstehenden Ansprüche, wobei das Verfahren für eine Zeitspanne von etwa 0,5 bis etwa 1 Stunde durchgeführt wird.

12. Verfahren nach einem der vorstehenden Ansprüche, wobei das Ammoniak in Form einer stark konzentrierten wäßrigen Ammoniaklösung bereitgestellt wird.

13. Verfahren nach einem der vorstehenden Ansprüche, wobei die Vorstufe und die Carboxypeptidase Y in Form einer wäßrigen ammoniakalischen Lösung bereitgestellt werden.

14. Verfahren nach einem der vorstehenden Ansprüche, wobei die Vorstufe, die Carboxypeptidase Y und das Ammoniak jeweils in Form einer wäßrigen Lösung bereitgestellt werden und die Lösungen in einem Verhältnis von etwa 1:1:8 vermischt werden.

15. Verfahren nach einem der vorstehenden Ansprüche, wobei das C-terminale amidierte Peptidprodukt eine Struktur aufweist, die mit der Struktur von humanem Calcitonin identisch ist oder eine mit humanem Calcitonin ausreichend ähnliche Struktur aufweist, so daß es die physiologische Aktivität von humanem Calcitonin besitzt.

## Revendications

1. Utilisation de la carboxypeptidase Y dans la préparation, à partir d'un précurseur, d'un produit peptidique ayant une proline amidifiée C-terminale, dans laquelle le précurseur possède une séquence peptidique supplémentaire constituée d'au moins deux restes d'acides aminés, non compris la proline, liés à la proline C-terminale et dans laquelle la séquence peptidique supplémentaire comprend un acide aminé hydrophobe lié à la proline par l'intermédiaire d'une liaison peptidique, ladite utilisation étant mise en oeuvre en présence d'ammoniac ou d'un agent libérant de l'ammoniac.

2. Procédé pour la préparation d'un produit peptidique amidifié sur sa proline C-terminale, comprenant la mise en contact d'un précurseur de ce produit avec la carboxypeptidase Y, en présence d'ammoniac ou d'un agent libérant de l'ammoniac pour digérer le précurseur, le précurseur possédant une séquence C-terminale d'au moins trois acides aminés débutant par la proline, la séquence ne comportant pas un autre reste proline, et dans lequel l'acide aminé adjacent à la proline est un acide aminé hydrophobe et est choisi de telle sorte que l'action de la carboxypeptidase Y sur cet acide aminé, après digestion du reste de la séquence, donnera la prolinamide.

3. Procédé pour la préparation d'un produit peptidique amidifié sur une proline C-terminale, comprenant le clivage d'une protéine de fusion génétiquement modifiée, précurseur de ce produit, pour donner un précurseur clivé du peptide amidifié sur son extrémité C-terminale et ensuite la mise en contact du précurseur clivé, avec la carboxypeptidase Y en présence d'ammoniac ou d'un agent libérant de l'ammoniac, pour donner le peptide amidifié sur son extrémité C-terminale, dans lequel le précurseur possède une séquence d'au moins deux acides aminés, autres que la proline, liée à son extrémité C-terminale, la séquence comprenant un acide aminé hydrophobe lié à la proline par l'intermédiaire d'une liaison peptidique.

4. Procédé pour la préparation d'un produit peptidique amidifié sur sa proline C-terminale, à partir d'un précurseur de ce produit, le précurseur comprenant un dérivé du peptide, dans lequel la proline C-terminale possède, à la place de l'amide, une séquence de deux ou de plus de deux acides aminés ayant la formule -Z-W, dans laquelle W représente un ou plusieurs acides aminés, les acides aminés étant identiques ou différents, mais ne comprenant pas la proline, le procédé comprenant la mise en contact du précurseur avec la carboxypeptidase Y en présence d'ammoniac ou d'un agent libérant de l'ammoniac, dans des conditions dans lesquelles la carboxypeptidase Y manifeste une activité de transpeptidase, pour ainsi cliver W, et dans laquelle Z représente un groupe choisi de telle sorte que l'action de la carboxypeptidase sur ce groupe, après clivage du groupe W, donnera la prolinamide C-terminale, ledit groupe comprenant un acide aminé hydrophobe lié à une proline C-terminale. par l'intermédiaire d'une liaison peptidique.

5. Procédé selon l'une quelconque des revendications précédentes, dans lequel, dans le précurseur, un acide aminé, choisi parmi la leucine, l'isoleucine, la valine, la phénylalanine et l'alanine, est lié à la proline par l'intermédiaire d'une liaison peptidique.

6. Procédé selon l'une quelconque des revendications précédentes, dans lequel l'ammoniac est présent en excès molaire.

7. Procédé selon l'une quelconque des revendications précédentes, dans lequel la carboxypeptidase Y est utilisée à une concentration finale d'environ 1 à environ 5 µM et dans lequel le précurseur est présent à une concentration finale d'environ 0,1 à environ 0,3 mM.

8. Procédé selon l'une quelconque des revendications précédentes, dans lequel le procédé est mis en oeuvre à un pH de 8,0 ou plus.

9. Procédé selon l'une quelconque des revendications précédentes, dans lequel le procédé est mis en oeuvre à une température comprise entre environ 25 et environ 55°C.

10. Procédé selon la revendication 9, dans lequel le procédé est mis en oeuvre à une température comprise entre environ 45 et environ 50°C.

11. Procédé selon l'une quelconque des revendications précédentes, dans lequel le procédé est mis en oeuvre sur une durée d'environ 0,5 à environ 1 heure.

12. Procédé selon l'une quelconque des revendications précédentes, dans lequel l'ammoniac est fourni sous la forme d'une solution aqueuse d'ammoniac très concentrée.

13. Procédé selon l'une quelconque des revendications précédentes, dans lequel le précurseur, et la carboxypeptidase Y sont fournis chacun sous la forme d'une solution ammoniacale aqueuse.

14. Procédé selon l'une quelconque des revendications précédentes, dans lequel le précurseur, la carboxypeptidase Y et l'ammoniac sont fournis chacun sous la forme d'une solution aqueuse et les solutions sont mélangées dans un rapport d'environ 1/1/8.

15. Procédé selon l'une quelconque des revendications précédentes, dans lequel le produit peptidique amidifié à son extrémité C-terminale possède une structure identique à celle de la calcitonine humaine ou possède une structure suffisamment similaire à celle de la calcitonine humaine pour posséder l'activité physiologique de la calcitonine humaine.
